# FASCICULE DE BREVET EUROPEEN

(11) **EP 0 523 153 B1**
(45) Date de publication et mention de la délivrance du brevet: **16.02.1994**
(21) Numéro de dépôt: 91907719.8
(22) Date de dépôt: 04.04.1991
(51) Int. Cl.: A61K 31/19, A61K 9/48

(54) **GELULE HUILEUSE DE KETOPROFENE**
ÖLIGES KETOPROFEN ENTHALTENDE KAPSEL
OLEAGINOUS CAPSULE OF KETOPROFEN

(30) Priorité: 06.04.1990 FR 9004468
(43) Date de publication de la demande: 20.01.1993
(73) Titulaire: RHONE-POULENC RORER S.A., 92160 Antony (FR)
(72) Inventeur: COURTEILLE, Frédéric, F-94230 Cachan (FR); VEILLARD, Michel, F-92330 Sceaux (FR)
(74) Mandataire: Le Pennec, Magali
(86) Numéro de dépôt international: FR9100273
(87) Numéro de publication internationale: WO9115203

(56) Documents cités:
- EP-A- 0 178 436
- EP-A- 0 349 509
- WO-A-89/04658
- Patent Abstracts of Japan, Vol. 6, No. 7, (C-87)(885), 16 January 1982 & JP, A, 56131514 (NISSAN KAGAKU KOGYO K.K.) 15 October 1981 see the whole document

## Description

La présente invention concerne une nouvelle forme pharmaceutique du kétoprofène. Elle concerne plus particulièrement une gélule contenant du kétoprofène en solution huileuse.

Le kétoprofène ou acide (benzoyl-3 phényl)-2 propionique est depuis longtemps commercialisé sous forme de comprimés à action immédiate ou à action prolongée et sous forme injectable, soit prête à l'emploi, soit lyophilisée. Lorsqu'il est commercialisé sous forme de comprimés on utilise la forme acide du kétoprofène, lorsqu'il est commercialisé sous forme injectable on utilise soit son sel de sodium, soit un sel azoté tel que le sel d'arginine et/ou de lysine et/ou de N-méthylglucamine.

Lorsqu'il est utilisé sous sa forme acide, donc par voie orale le kétoprofène a tendance lors de son absorption à provoquer des irritations gastriques ou duodénales par contact des cristaux de principe actif avec les muqueuses qui entraine une concentration locale en principe actif très élevée.

Lorsque l'industrie pharmaceutique a cherché à préparer des formes destinées à l'absorption par voie orale à partir d'un des sels du kétoprofène, il est apparu de grosses difficultés de stabilité des comprimés ainsi préparés car les sels de kétoprofène présentent une telle hygroscopicité que le comprimé a tendance à se dégrader rapidement ou oblige à maintenir ceux ci dans un conditionnement présentant une atmosphère strictement contrôlée en ce qui concerne l'hygrométrie.

Les formes injectables ne présentent pas cet inconvénient car soit elles sont présentées en flacons hermétiques soit elles sont en solution aqueuse et ne présentent donc pas de problèmes liés aux phénomènes d'hygrométrie.

Afin d'éviter le contact direct de la muqueuse gastrique ou duodénale avec la forme acide du kétoprofène on cherche depuis longtemps une forme d'administration orale du kètoprofène sous forme saline.

La présente invention a permis d'atteindre cet objectif.

Elle concerne des gélules contenant une solution huileuse de kétoprofène. Elle a pour objet des gélules caractérisées en ce qu'elles contiennent une solution huileuse d'un sel de kétoprofène, la solution huileuse étant composée d'huile végétale polyoxyéthylénée, d'huile de ricin ou d'esters d'acides gras ou de polyols.

Cette solution est obtenue par dissolution d'un sel de kétoprofène choisi parmi le sel de sodium, d'arginine et/ou de lysine et/ou de N-méthylglucamine dans une huile végétale polyoxyéthylénée, un ester d'acide gras et d'un alcool ou d'un polyol ou l'huile de ricin. Parmi les huiles végétales polyoxyéthylénées on peut citer les huiles vendues par GATTEFOSSE sous la marque LABRAFIL.

Après de nombreux essais de solubilisation du sel de sodium dans des compositions huileuses différentes, il est apparu que l'huile végétale polyoxyéthylénée vendue sous la dénomination LABRAFIL permettait le meilleur rapport économie, tolérance et solubilité pour le kétoprofène.

Il est apparu que les huiles polyoxyéthylénées possédaient des propriétés de solubilité encore améliorées si elles étaient associées avec un ou plusieurs tiers corps tel que par exemple: le polyéthylène glycol 400, l'oléate d'éthyle, le Crémophor EL, l'éthanol, les agents émulsifiants comme les esters de sorbitane et de polyoxyéthylène (Tween 80), l'huile neutre composée de triglycérides d'acides gras saturés de longueur de chaîne moyenne (8 à 10 atomes de carbone) (le Miglyol 812), l'huile de maïs.

On préfère parmi toutes ces associations utiliser les huiles polyoxyéthylénées associées avec le polyéthylène glycol 400 ou avec un mélange d'éthanol et de polysorbate 80 (Tween 80). Il faut spécifier que dans toutes ces associations on utilise au moins 90 % d'huile.

Les compositions pharmaceutiques préférées selon l'invention contiennent entre :
- 1 à 8 % en poids de kétoprofène sodique
- 94 à 77 % en poids de Labrafil M.1944
- 5 à 15 % en poids d'éthanol à 95 ou d'un mélange 50/50 d'éthanol et de polysorbate 80 (Tween 80).

Cette solution est préparée par mélange des composants de l'excipient, suivi d'une incorparation lente du kétoprofène sodique et agitation jusqu'à dissolution complète du kétoprofène sodique. La division et la répartition est effectuée dans des gélules qui sont scellées par toute technique appropriée comme par exemple au moyen d'une banderole de gélatine (technique Eli Lilly) ou pulvérisation et séchage d'un mélange d'eau et d'alcool (technique Capsugel).

La présente invention sera plus complètement décrite à l'aide des exemples suivants qui ne doivent pas être considérés comme limitatifs de l'invention.

### EXEMPLE 1

On mélange à l'abri de la lumière 3749,85 g de LABRAFIL M.1944 CS avec 450 g d'éthanol à 95°. Dans un récipient annexe on incorpore, sous agitation, 300,15 g de kètoprofènate de sodium dans 30 % environ du mélange précédent. On coule ensuite le reste de l'excipient et on maintient sous agitation jusqu'à dissolution totale du kétoprofène (environ 5 heures). On ajuste le poids final de la préparation pour compenser les pertes en éthanol. On filtre sur filtre Millipore de 10 _m de porosité.

La division en gélules est effectuée à l'aide d'une machine GKF 400L HOFLIGER et KARG, à raison de 0,43ml de solution par gélule pour une gélule n° 3. On obtient environ 9000 gélules. Les gélules pleines sont serties par une banderole de gélatine à l'aide d'une machine Quali-seal sous une pression de 200 mPas. Les gélules sont ensuite séchées à l'abri de la lumière.

Après analyse, on obtient un titre en kétoprofène par gélule de 24,5 mg pour un titre théorique de 25 mg en kétoprofène acide.

### EXEMPLE 2

On reproduit l'exemple 1 en utilisant comme excipient à la place des 450 g d'éthanol 225 g d'éthanol à 95° et 225 g de polysorbate 80. La préparation de la solution et la répartition en gélules sont effectuées comme à l'exemple précédent.

Après analyse on obtient un titre en kétoprofène par gélule de 24,8 mg pour un titre théorique de 25 mg en kétoprofène base acide.

### EXEMPLE 3 ET 4

On reproduit les exemples 1 et 2 en utilisant 600,30 g de kétoprofènate de sodium que l'on dissout dans respectivement 7500 g de LABRAFIL avec 900 g d'éthanol ou 450 g d'éthanol et 450 g de polysorbate 80 que l'on répartit dans des gelules n° 0 (1 ml).

On obtient des gélules contenant l'équivalent de 50 mg en en kétoprofène acide.

## Revendications

1. Gélules caractérisées en ce qu'elles contiennent une solution huileuse d'un sel de kétoprofène, la solution huileuse étant composée d'huile végétale polyoxyéthylénée, d'huile de ricin ou d'esters d'acides gras ou de polyols.

2. Gélules selon la revendication 1 caractérisées en ce que le sel est choisi parmi le sel de sodium, le sel d'arginine ou le sel de lysine et/ou de N-méthylglucamine.

3. Gélules selon la revendication 1 caractérisées en ce qu'elles contiennent une concentration pondérale en sel de kétoprofène de 5 % exprimé en kétoprofène acide.

4. Gélules selon la revendication 1 caractérisées en ce qu'elles contiennent de l'éthanol.

5. Gélules selon la revendication 1 caractérisées en ce qu'elles contiennent un agent émulsifiant.

6. Gélules selon la revendication 5 caractérisées en ce que l'agent émulsifiant est un ester de sorbitol et de polyoxyéthylène.

## Patentansprüche

1. Kapseln, dadurch gekennzeichnet, daß sie eine ölige Lösung eines Salzes von Ketoprofen enthalten, wobei die ölige Lösung zusammengesetzt ist aus polyoxyethyleniertem Pflanzenöl, Ricinusöl oder Estern von Fettsäuren oder Polyolen.

2. Kapseln nach Anspruch 1, dadurch gekennzeichnet, daß das Salz unter dem Salz von Natrium, dem Salz von Arginin oder dem Salz von Lysin und/oder von N-Methylglucamin ausgewählt wird.

3. Kapseln nach Anspruch 1, dadurch gekennzeichnet, daß sie eine Gewichtskonzentration an Ketoprofensalz von 5 % enthalten, ausgedrückt als saures Ketoprofen.

4. Kapseln nach Anspruch 1, dadurch gekennzeichnet, daß sie Ethanol enthalten.

5. Kapseln nach Anspruch 1, dadurch gekennzeichnet, daß sie einen Emulgator enthalten.

6. Kapseln nach Anspruch 5, dadurch gekennzeichnet, daß der Emulgator ein Ester von Sorbitol und Polyoxyethylen ist.

## Claims

1. Gelatin capsules characterized in that they contain an oily solution of a ketoprofen salt, the oily solution being composed of polyoxyethylenated vegetable oil, castor oil or esters of fatty acids or polyols.

2. Gelatin capsules according to claim 1, characterized in that the salt is chosen from sodium salt, arginine salt or lysine and/or N-methylglucamine salt.

3. Gelatin capsules according to claim 1, characterized in that they contain a ketoprofen salt concentration by weight of 5 % expressed in terms of acidic ketoprofen.

4. Gelatin capsules according to claim 1, characterized in that they contain ethanol.

5. Gelatin capsules according to claim 1, characterized in that they contain an emulsifying agent.

6. Gelatin capsules according to claim 5, characterized in that the emulsifying agent is an ester of sorbitol and polyoxyethylene.
